Europäisches Patentamt

European Patent Office

Office européen des brevets

(19)

(11) Publication number: **0 269 388 A2**

(12) **EUROPEAN PATENT APPLICATION**

(21) Application number: 87310282.6

(22) Date of filing: 20.11.87

(51) Int. Cl.⁴: **G 01 N 33/569**
G 01 N 33/577
// C12N5/00, C12N15/00,
C12P21/00

(30) Priority: 20.11.86 US 933039

(43) Date of publication of application:
01.06.88 Bulletin 88/22

(84) Designated Contracting States:
BE CH DE ES FR GB IT LI NL SE

(71) Applicant: MINNESOTA MINING AND MANUFACTURING
COMPANY
3M Center, P.O. Box 33427
St. Paul, Minnesota 55133-3427 (US)

(72) Inventor: Shelburne, Charles E. c/o Minnesota Mining
and
Manufacturing Comp. 2501 Hudson Road PO. Box 33427
St. Paul Minnesota 55133-3427 (US)

(74) Representative: Baillie, Iain Cameron et al
c/o Ladas & Parry Isartorplatz 5
D-8000 München 2 (DE)

(54) Method for the evaluation of oral microbes.

(57) The invention relates to a method for the evaluation of oral microbes associated with periodontal disease, and to antibodies, including monoclonal antibodies useful in such a method, as well as to hybrid cell lines for the production of the monoclonal antibodies. The method results in a comparison of one or more specific oral microbes to a reference group of gram-negative oral microbes.

EP 0 269 388 A2

**Description**

## METHOD FOR THE EVALUATION OF ORAL MICROBES

TECHNICAL FIELD

This invention relates to methods for the evaluation of oral samples for the presence of oral microbes in order to monitor microbially-mediated periodontal diseases. In another aspect this invention relates to materials suitable for use in such methods.

BACKGROUND ART

Periodontal disease is a general term used to describe a variety of conditions in which the supporting tissue surrounding the teeth is inflamed and/or diseased. Most affected individuals are first afflicted with a form of periodontal disease known as gingivitis, which is commonly a mild, chronic, reversible inflammation of the gingival tissue. In its more severe forms, the disease process can be so extensive that adjacent gingival tissue is destroyed, and a pocket forms around the tooth. In time, this could result in advanced pocket depth, vertical bone loss and the loss of the tooth.

A major advance in the understanding of periodontal disease has been the realization in recent years that many forms of the disease are microbially-mediated conditions, and therefore that it may be possible to distinguish healthy from diseased individuals, and to distinguish between diseased sites within an individual, by assessing the microbial flora at those sites. Various microbes have been associated with such forms of periodontal disease as gingivitis, juvenile periodontosis, adult periodontitis, and adult necrotizing and ulcerative gingivitis. See, generally, R.C. Page, J. Clin. Periodontol. 13:345-355 (1986) and D.H. Fine et al., J. Clin. Periodontol. 13:533-546 (1986).

To date, the precise etiologic or pathogenic role, if any, of any particular microbial species has not been fully elucidated. Neither have researchers yet fully defined the interrelationship of various species to each other or to the changing oral environment. In large part this lack of knowledge is due to the difficulty encountered with the use of traditional techniques to identify systematically, accurately and reproducibly the various species and subspecies that may be involved.

This difficulty arises largely from the fact that the microbes of interest can often only be cultured, if at all, by means of complex and laborious strict anaerobic culture techniques. Moreover the oral sample, e.g., plaque, from which these microbes are generally isolated is a difficult specimen for microbiological analyses since it is subject to error and problems in its collection and preparation (e.g., maintaining sterility and anaerobicity), and frequently can contain substances that may interfere with certain traditional analyses.

The many species that have been implicated as being associated with periodontal disease are most often detected (and sometimes differentiated) by techniques such as anaerobic culture methods, dark-field microscopy, and more recently, by phase contrast microscopy. Suggested methodologies for the evaluation of the microbes themselves have also included flow cytometry and DNA probes. Other approaches for the evaluation of the microbial flora include the determination of microbial products, host cell responses and products, immune responses, and intracellular enzymes.

Traditional immunologic techniques have also been used, such as fluorescent-antibody microscopy, which involve the production of antibodies against certain oral microbes (see e.g., Jacob et al, J. Clin. Microbiol., 10:934- 936 (1979)). These techniques, though appropriate for research purposes, are generally too laborious, time-consuming and expensive for the rapid and routine clinical evaluation of numerous sites.

In recent years monoclonal antibody technology has grown to allow the production of antibodies specific for a variety of microbes and microbial antigenic determinants. See, e.g., Mutharia et al., "Use of Monoclonal Antigens in the Study of Common Antigens of Gram-Negative Bacteria", in Monoclonal Antibodies Against Bacteria, 6:131-142, Acad. Press, (1985) and Macario et al., American Society of Microbiology News 49:1-7 (1983). Still, the use of this technology for the investigation of microbial pathogenesis has only recently been explored. Monoclonal antibodies have been reported, for instance, against some oral Bacteroides species (see e.g., Ebersole et al., J. Clin. Microbiol., 19:639-644 (1984)).

Monoclonal antibodies have purportedly been produced as well against the endotoxin core of the lipopolysaccharide of gram-negative bacteria, a structure which is said to be highly analogous and immunologically cross-reactive between most gram-negative bacteria (see, e.g., published PCT application no. WO84/04458). Yet another research group, however, was unable to isolate an antibody showing cross reactivity to a group of three gram-negative oral species. (see Dahlen et al., Inf. Immun. 39:466-468 (1983)).

Another reason why the precise role of any particular species has not been clearly elucidated, and why, in turn, the evaluation of the microbial flora is not commonly used to monitor the disease, is because of the significant problems of data interpretation that have historically been encountered. In view of differing experimental approaches and protocols, and the sheer variability of the disease itself between sites and stages as well as individuals, no method has to date been shown to be simple, repetitive, specific, and effective enough to become a widely acceptable means of establishing a usable relationship between the presence or amount of any particular species and the stage, course or severity of disease.

Unlike more common microbially-mediated diseases where the mere presence of a particular organism very often can be used to diagnose and/or monitor the associated disease, the oral species associated with periodontal disease are numerous, varied, and most importantly, frequently part of the normal flora of the mouth as well as part of the diseased flora.

The numbers and types of the oral species seem to vary independently in heretofore unpredictable and bewildering ways, both between themselves (e.g., over time), as well as between any particular species and the presence or stage of disease. Furthermore, the disease is a chronic one in which changes in the flora progress very slowly over time, and in turn manifest themselves even more slowly, if at all, in clinical symptoms.

Hence there remains an almost exclusive reliance on the evaluation of periodontal disease by the use of standard clinical, anatomic and morphologic findings, looking at parameters such as plaque index, probing depth, attachment level, gingival coloration, bleeding tendency and the like. These methods measure the symptoms of the disease however, and are relatively subjective. Furthermore, they are not very useful for determining the progress or pattern of disease activity, e.g., its exacerbation or remission at individual sites, or for concurrently assessing the effectiveness of therapeutic measures.

## SUMMARY OF THE INVENTION

The study of periodontal disease, and therefore its diagnosis and treatment, has lagged behind that of many other microbially-mediated diseases, in large part for want of the tools and interpretative means necessary for the rapid, reproducible and accurate analysis of the microbial flora. The present invention provides such a tool.

In summary, the invention provides a method for evaluating an oral sample for the presence of microbial flora associated with periodontal disease comprising the steps of:

(a) determining, by the use of antibodies, the amount in said sample of one or more specific oral microbe(s),

(b) determining, by the use of antibodies, the amount in said sample of a reference group of gram-negative oral microbes, and

(c) comparing the amount of said specific oral microbe(s) to the amount of said reference microbes.

In another aspect the present invention provides antibodies, and kits comprising these antibodies, useful in carrying out the method of the invention, and also provides hybridoma cell lines producing such antibodies.

## DETAILED DESCRIPTION OF THE INVENTION

This method allows the evaluation of the microbial flora in an oral sample such as plaque or gingival fluid, in a manner that is rapid, precise, simple and yields significant and useful results, i.e., the results correlate well with those obtained by the longer and more laborious process of anaerobic cultivation of the microbes. By employing antibodies, this method offers significant advantages over more traditional approaches, such as those requiring cultivating, staining or otherwise identifying such microbes through laborious or expensive techniques. By providing a comparison of individual microbes to a reference group of gram-negative microbes, rather than merely to other individual microbes, the method gives more accurate and/or dramatic evaluation results than are obtained if individual microbes are merely compared to themselves (e.g., over time or between sites), or to a larger group that includes all identifiable or cultivable microbes (e.g., including gram-positive microbes).

The term "periodontal disease" as used herein refers to any microbially-mediated or -associated inflammation, disease or other condition at any stage or site in the oral environment of mammals.

The term ""oral microbe" as used herein refers to any of the various microbial orders, families, genera, species, subspecies and strains, and products thereof, that can exist in the mouth during the development and course of periodontal disease, whether the particular microbe is itself a causative agent of the disease or not.

The word "amount" as used herein refers to a determination that is sufficiently quantitative and/or semiquantitative for its intended purpose, e.g., quantitative as in the determination of the concentration of an oral microbe or group of oral microbes, or semiquantitative as in the sense of a comparative determination showing the existence of the oral microbe or group at a level above or below a predetermined level.

The term "specific oral microbe" as used herein refers to one or more genera, species, subspecies or strains of oral microbes capable of being determined by a single antibody.

The word "battery" as used herein refers to a group of two or more specific oral microbes.

The word "reference" as used herein refers to one or more genera of gram-negative oral microbes, and can include all gram-negative oral microbes, or subsets thereof. The group of reference microbes determined in step (b) however, should not be identical to any specific oral microbes determined in step (a) of the method of the invention, i.e., the reference microbes should include at least some microbes different from any particular determination in step (a). Preferably the presence of such reference microbes can be simultaneously determined, as by the use of a single antibody. Preferably also, the reference microbes include each of the specific oral microbes determined in step (a) of the method of the invention.

In a preferred embodiment the specific oral microbes determined in step (a) are gram-negative oral microbes, and most preferably those belonging to the following genera: Actinobacillus, Bacteroides, Fusobacterium, Haemophilus, and Treponema. In a particularly preferred embodiment the amount of each specific oral microbe in a battery is determined, the battery including two or more of the following gram-negative oral microbial species: Actinobacillus actinomycetumcommitans, Bacteroides gingivalis, Bacteroides intermedius, Fusobacterium nucleatum, Haemophilus aphrophilus, Treponema denticola and Treponema vincentii. Further preferred is the quantitative determination of these specific oral microbes by the use of monoclonal antibodies specific for antigens provided by the lipopolysaccharide ("LPS") component of each of these microbes.

A preferred method of this invention also involves the comparison of the amount of these specific oral microbes to the amount of reference microbes by the use of monoclonal antibodies specific for an antigen provided by an LPS component that is shared by all such reference microbes.

The invention takes advantage of the existence of antigens that enable discrimination between specific oral microbes and reference microbes. Antibodies can be prepared to detect such antigens, the resultant antibodies being useful in an assay for the presence of such antigens in the complex environment that typifies oral samples such as plaque and gingival fluid. Through the use of the invention the amount of individual specific oral microbes can be determined in an oral sample concurrently with the determination of the amount of reference microbes and can be compared in a useful and significant manner.

The following discussion will set forth considerations helpful in making materials useful in performing the method of this invention.

The preferred method of the present invention requires, at a minimum, an antibody for a specific oral microbe and an antibody for reference microbes.

The word "antibody" as used herein refers to any antibody that is sufficiently discriminating to enable its use in the method of the invention, e.g., monospecific polyclonal antibodies as well as monoclonal antibodies. Preferred antibodies for use in the method of the present invention are monoclonal antibodies.

While any immunoglobulin can be employed, IgG is preferred. Either whole antibodies or fragments thereof can be employed. Single monoclonal antibodies can be employed, or mixtures thereof, including mixtures of monoclonal antibodies or mixtures of polyclonal antibodies. The number and type of antibodies that are employed will depend upon the antigenic site and number of different antigens that are to be detected. The antibody composition preferably is free of non-specific antibodies, i.e., antibodies that bind to antigens other than the desired antigens.

The antibodies useful in the present invention can be obtained by a variety of methods that will become apparent to those skilled in the art, including in vitro stimulation of lymphocytes with mitogens and/or antigens, splenic fragment culture, virus transformation of lymphocyte clones or recombinant DNA techniques.

Monoclonal antibodies are preferably obtained by a process for the production of hybridomas similar to that discussed by Milstein and Kohler and reported in Nature, 256: 495-497, 1975. This process involves injecting a mouse (or other suitable animal) with the desired immunogenic material. In the present invention that material can be a culture of partially or completely purified oral microbes. That material can also be a purified or partially purified component of the organisms, such as enzymes or toxins, or LPS which has been purified, e.g., by the sonication and extraction method described in WO84/04458, or other methods cited therein, the disclosure of which is hereby incorporated by reference.

To prepare monoclonal antibodies, suitable hosts such as mice are immunized with potential antigen-containing suspensions of cells or cell fractions according to immunization methods, routes and schedules generally in keeping with established techniques for antibody stimulation and production. After immunization the immune lymphoid cells are isolated, preferably from the spleens, and are fused with suitable myeloma, plasmacytoma or hybridoma cells to generate hybrid cell lines (i.e., hybridomas) which produce monoclonal antibodies to the antigens of choice and which can be cultivated and subcultivated indefinitely.

The population of hybridomas formed by fusion can then be screened for immunoglobulin production. Any of the several known methods for screening for immunoglobulins can be used, such as enzyme-linked immunoassay (ELISA) using purified antigen, according to known techniques. The immunoglobulins present in the cell culture fluids are further examined for their ability to react with the microbial cells or fractions used for immunization. This can be accomplished by modifying the above mentioned immunoassay in a fashion known to the art.

If hybridoma cultures are found to produce monoclonal antibodies that react with the immunizing antigen they are further examined to determine their specificity. The cultures are cloned by limiting dilution to assure monoclonality using techniques well known to those skilled in the art, e.g., Thomas J. McKearn, "Cloning of Hybridoma Cells by Limiting Dilution in the Fluid Phase" in Monoclonal Antibodies, p. 374,, Kennett, R. H., McKearn, T. J. and K. B. Bechtol, eds. Plenum Press, N.Y. (1980).

The monoclonal antibodies produced by these cloned hybridomas are tested to determine their cross-reactivity with antigens other than those of the immunizing strain or antigen by an ELISA assay. In addition, the antibodies are assayed for their ability to bind microbial cell components separated by electrophoretic means (e.g., "Western Blots" and immunoprecipitation) known to those skilled in the art (see e.g., Tobin, et al. Proc. Nat. Acad. Sci. 76:4350-4354 (1979)).

If any hybridoma cell lines are found to produce monoclonal antibodies with the desired specificity, they can then be cultivated and subcultivated to establish continuous production of antibodies, e.g., by traditional tissue culture of mouse ascites fluid production techniques well known to those skilled in the art. These cell lines can be stored and preserved by conventional techniques, including lyophilization or freezing.

The antibodies used in the assay are isolated by conventional techniques such as ion exchange chromatography or precipitation, e.g., by treating tissue culture fluid or clarified ascites fluid with 50% ammonium sulfate. This treatment results in the precipitation of antibodies. The precipitate is optionally (and preferably) resuspended in a buffered saline solution (phosphate buffered saline, "PBS", pH 7.5) for further use. Recovered antibodies can be stored, e.g., lyophilized, frozen or refrigerated according to known techniques.

Antigens suitable for use in the method of the present invention include any cellular or extracellular molecular species that allow one to distinguish between the specific oral microbe(s) and the reference microbes, and preferably allows one to further discriminate between individual specific oral microbes.

Cellular components or products suitable for providing antigenic sites (i.e., epitopes), include any that are capable of serving as antigens, i.e., are immunogenic alone or as haptens, and that are capable of being immunologically reactive with monoclonal antibodies in oral samples evaluated according to the methods of the invention.

Suitable antigens include those located in, e.g., the flagella, cell envelope, capsule, or cytoplasm, or antigens produced by the oral microbe and found in its extracellular environment such as extracellular factors, toxins, or enzymes.

Preferred antigens are those showing optimal specificity and binding characteristics for the microbe or group of choice, as well as those antigens that are detectable with minimal physical manipulation of the sample or cell, e.g., extracellular or easily accessible cell wall or envelope antigens. Particularly preferred are antigens that are provided by the same or similar molecular components, such that direct comparisons can be made safe in the assumption that the antigens will be similarly affected by the handling, extraction and assay procedures chosen.

A preferred antigenic source is lipopolysaccharide ("LPS"). For a review of LPS see, generally, R.J. Elin et al., CRC Handbook of Microbiology Laskin et al., eds., Vol. II, 215-239 (1973), and I.W. Sutherland, Ann. Rev. Microbiol. 39:243-270 (1985).

LPS is an ideal source of antigens for a variety of reasons. LPS is a cell wall constituent that is present in large amounts in most if not all gram-negative oral microbes. Applicant has been able to identify LPS antigens in species in which the presence of LPS has heretofore been unknown or debated, e.g., in T. vincentii. Moreover, LPS is made up of both relatively "common" regions (i.e., the core and lipid A regions) that can serve as the source of antigens for reference microbes in the method of this invention, as well as relatively "variable" regions (i.e., the "O antigen" region) that can serve as the source of antigens to discriminate between specific oral microbes and reference microbes.

Moreover, LPS appears to exhibit a high degree of resistance, in both its common and variable regions, to change caused by many physico-chemical treatments employed in its extraction and preparation for assay.

Yet another advantage of the use of LPS is that, as an endotoxin as well as cell wall component, some LPS can be expected to be found in an oral sample in an extracellular form as well as in its cellular form see, e.g., C.G. Daly et al., J. Oral Pathol. 9:1-15 (1980). As a result, there exists a larger detectable pool of LPS than may be the case for antigens that are present only extracellularly or only in the cellular form. The ability to detect simultaneously both forms of LPS as disclosed herein provides, in a sense, the best features of both an endotoxin assay and a whole cell assay.

The method of the present invention employs specific oral microbes, preferably in the form of a battery. Suitable specific oral microbes include detectable genera, species, subspecies and strains of oral microbes.

Specific oral microbes suitable for use in the method of the present invention include, but are not limited to microbes from one or more of the following genera:

| | |
|---|---|
| Actinobacillus | Lactobacillus |
| Actinomyces | Leptotrichia |
| Arachnia | Peptococcus |
| Bacteroides | Peptostreptococcus |
| Bacillus | Propionibacterium |
| Bixidobacterium | Rothia |
| Capnocytophaga | Selenomonas |
| Desulformonas | Streptococcus |
| Enbacterium | Treponema |
| Fusobacterium | Veillonella |
| Haemophilus | |

Preferred are specific oral microbes that vary rapidly and appreciably in numbers well in advance of any concomitant physical changes associated with periodontal disease. Preferred also are specific oral microbes that actually play a role in the pathogenic process of periodontal disease, as opposed to those merely affected by it. Further preferred are gram-negative specific oral microbes. Preferably a battery consists of two to twenty, and more preferably two to ten specific oral microbes.

Suitable reference microbes include any combination of gram-negative oral microbes that are detectable as a group (e.g., all gram-negative oral microbes or subsets thereof including individual or multiple families, orders or genera that provide a meaningful comparison in step (c) of the method of this invention.

Preferred reference microbes can be determined by means of a single antibody. As noted above, reference microbes preferably include each of the specific oral microbes determined in step (a) of the method of the invention.

Particularly preferred reference microbes are all gram-negative oral microbes, as detected by a single antibody.

Antibodies to specific oral microbes and reference microbes can be made into a kit to facilitate their use in a variety of immunoassay formats known to those skilled in the art. Preferably the antibodies within a particular kit are monoclonal antibodies specific for antigens provided by a single molecular component of the specific oral microbes and reference microbes. Most preferably that molecular component is LPS.

The particular immunoassay format in which the presence of specific oral microbes and reference microbes is determined is not critical in this invention, so long as the format provides the desired degree of sensitivity and reliability. A number of different types of assays exist having a variety of protocols and labels. For the most part, the commonly available assays for detecting specific determinant sites are competitive protein binding assays, in which antibodies or fragments thereof are employed. As illustrative of the various assays, see U.S. Pat. Nos. 3,654,090, 3,817,837, 4,233,402, 4,275,149 and 4,584,268. Generally, a reagent solution is formed containing labeled antibody or labeled antigen. The reagent solution can contain, in addition to the labeled component, other additives, such as buffers, e.g., phosphate, tris, barbital, or the like, normally at concentrations in the range of about 0.01 to about 10 mM, the concentration being sufficient to maintain a pH in the range of about 6 to about 9, more usually 7 to 8 during the assay. Other additives include preservatives, e.g., sodium azide, inert protein, e.g., serum albumin, sodium chloride, detergents, or the like, which aid in preserving the labeled component, enhancing the formation of the antigen-antibody complex, preventing non-specific binding, or the like.

The following discussion considers the use of materials such as those described above in order to perform the method of the present invention. Ideally the assays are dispensed in a fashion that enables their use in a quick and easy fashion by dental personnel, providing usable results without undue time, expense or instrumentation. In a preferred embodiment the method of the present invention can be used in the following manner:

Oral samples can be obtained by any means, and from any source or location that enables one to react immunologically the antigen(s) of interest. Samples need not contain the oral microbes themselves, for instance, if the antigen is found extracellularly. Samples can be taken directly from the oral environment, but can also be artificially prepared, e.g., from cultivation or preservation media, from which oral microbes can be purified or otherwise detected.

In the oral environment samples can be physically obtained, for instance, from saliva, the tongue or tooth surfaces, supragingival plaque and subgingival plaque.

Studies indicate that the preferred oral sites for sample collection will be gingival crevices and subgingival plaque. Samples can also be assayed in vivo, e.g., by using monoclonal antibodies to detect, localize or otherwise react immunologically with oral microbes in the oral environment.

Oral samples can be obtained by a variety of means known to the art, including the use of a sterile periodontal sealer, gas-flushed syringe, filter paper strip, glass cuvette or pipette and so on. It is generally not necessary to the practice of this invention to maintain anaerobic, or even sterile conditions since the microbes in samples generally need not be isolated or cultivated. Samples can be analyzed concurrently with sampling or can be stored according to methods known in the art.

As noted above, the sample is evaluated in order to determine the amount of one or more specific oral microbes and compare that determination to the amount of reference microbes. Steps (a) and (b) of the method of the invention can be performed in either order, and are preferably performed concurrently using aliquots of the same oral sample.

The results of the comparison can be presented using a variety of techniques. For instance, the results can be expressed in the form of a ratio or ratios comparing the amount of specific oral microbe(s) to the amount of reference microbes. Such ratios can be used in conjunction with instructions, e.g., standard curves, to correlate the ratio with disease. The ratio also can be calculated as well as interpreted by means of computer-aided analysis to indicate a relationship with periodontal disease.

The results of this invention need not be presented solely in the form of a ratio. The results can, if desired, be determined and presented in a form as simple as a plus or minus indication of whether a particular comparison is within a particular limit or range. Similarly, other valid comparisons can be in the form, for instance, of a multivariant assay analysis.

It is also possible to correlate the results obtained by the method of this invention with the results of other observations or measurements concerning the sample (such as probing depth, microscopic evaluations, protein determinations, and live cell counts) or concerning the patient, such as the clinical parameters discussed above, as well as immune response, temperature, nutritional state, hygiene and so on. In this way periodontal disease and its treatment can be better defined and understood and more closely followed.

Kits prepared using the antibodies of the present invention can include the antibodies alone, or may include a variety of options for packaging of devices and equipment, including filters and reagents, which are currently commercially available and known to those skilled in the art. Useful optional ingredients are second, enzyme-linked antibodies to the specific oral microbe antibodies and reference microbe antibodies of the assay, which will serve as a part of the detection system, as well as buffers, enzyme substrates and the like.

Antibodies labeled with fluorescent compounds or radioisotopes could also be used in a modification of this assay.

The following examples are offered to aid understanding of the present invention and are not to be construed as limiting the scope thereof. Unless otherwise indicated, all percentages are by weight, and the volumes of monoclonal antibodies added represent the volume of recovered ascites fluids as diluted with phosphate buffered saline in order to achieve a concentration of approximately 100 μg/ml.

Example 1

Production of Monoclonal Antibodies to Intact Treponema denticola and Treponema vincentii.

The production of monoclonal antibodies to two oral spirochete species, T. denticola and T. vincentii, will be described in order to illustrate certain considerations involved in the preparation of discriminating monoclonal antibodies to two closely related oral microbial species.

Pure cultures of T. denticola or T. vincentii were obtained from American Type Culture Collection, Rockwell (ATCC 3520) and Dr. R.C. Johnson, University of Minnesota, respectively. The T. vincentii cultures (and other wild type Treponeme strains described below) were isolated and identified according to the method described in R.C. Johnson, Ann. Rev. Microbiol. 31:89-106 (1977), and grown as described by R.M. Smibert, in The Prokaryotes (M.P. Starr, et al., eds.) pp. 564-577, Springer-Verlag Berlin Heidelberg (1981), the disclosures of which are both hereby incorporated by reference. The cells were separated from their culture medium by gradient centrifugation in colloidal polyvinylpyrrolidine-coated silica ("Percoll", Pharmacia Fine Chemicals, Piscataway, N.J.). Each culture was diluted with 90% Percoll in phosphate buffered saline ("PBS", pH 7.4) (0.39 ml Percoll/ml culture) and centrifuged at 31,000 × g for 20 minutes at 4°C. The band of spirochetes was recovered with a pipette and recentrifuged at 100,000 × g for 1 hour at 4°C to reinforce the Percoll. The pelleted spirochetes were resuspended in PBS at $10^8$ cells/ml and frozen at -70°C.

To immunize mice with T. denticola, 5-week old female balb/C mice (Chas. Rivers, Wilmington, MA) were immunized with 5 × $10^6$ washed spirochetes in PBS by intraperitoneal injection. Over a period of 2 months the animals were injected six times. Four days prior to fusion the mice were injected with $10^6$ spirochetes via the tail vein. A similar immunization schedule was used with T. vincentii.

To obtain hybridomas producing antibodies to T. denticola the spleens of three immunized mice were removed and processed to isolate the leukocytes. The leukocytes were combined with the NS-1 drug-marked mouse myeloma (available from ATCC-T1B18) at a ratio of 4 spleen cells for each myeloma cell and fused in 35% polyethylene glycol using the fusion technique described in Galfre et al., Nature 266:550-552 (1977) the disclosure of which is hereby incorporated by reference. The resultant cell suspension was cloned in tissue culture medium (Dulbecco's Modified Eagles Medium (Sigma Chemical Co., St. Louis, MO) with 4500 mg/l glucose and 20mM N-2-hydroxyethylpiperazine-N'-2-ethanesulfonic acid ("HEPES", Sigma Chemical Co., St. Louis, MO) supplemented with HAT (0.136 mg/100 ml hypoxanthine, 0.0018 mg/100 ml aminopterin, and 0.076 mg/100 ml thymidine, pH 7.2-7.4, all commercially available from Sigma Chemical Co.). In this medium unfused myeloma cells are selectively killed, allowing outgrowth only of the hybrid cells.

After 10 days of growth, cultures with hybridomas were tested for production of antibody to T. denticola by an ELISA immunoassay as follows.

$10^6$ Spirochetes (T. denticola) were crosslinked to the bottom of microtiter plates (Immulon II, Dynatech, Alexandria, Va.) using glutaraldehyde, by first coating the plates with 0.1% glutaraldehyde in water for 1 hour, then rinsing the plates 3 times in water. Spirochetes were then added in 100µl PBS, and incubated for 2 hours at room temperature. The plates were then blocked with 1% bovine serum albumin (Sigma Chemical Co., St. Louis, MO) and washed 3 times with PBS. Supernatants (50µl) from hybridoma cultures were added for one hour at room temperature, then removed and the wells rinsed with PBS. 100 ml peroxidase-labeled goat anti-mouse immunoglobulin (50 ng/ml) (Kirkegarrd and Perry Laboratories, Gaithersburg, MD) was added and the mixture incubated for an additional hour at room temperature. The unbound antibody was removed, the plates washed as before and 100µl of peroxidase substrate solution (orthophenylenediamine (Sigma Chemical Co.), 50 mg/100 ml; $H_2O_2$, 0.03%, citrate buffer, pH 4.5) added. The reaction was stopped by the addition of 50µl of 2.5 M $H_2SO_4$ after 10 minutes and the optical density (490 nm) of each well was determined spectrophotometrically. Hybridoma cultures represented by positive assay results were chosen for expansion and the rest discarded.

Hybridomas producing antibodies to T. vincentii were constructed and screened in a similar manner, except the SP2/0 mouse myeloma (ATCC-CRL1581) was used in place of NS-1, and cultures were screened using T. vincentii immobilized on the microtiter plates. Again, cultures showing positive assay results were chosen for expansion and the rest discarded.

Example 2

Specificity of Monoclonal Antibodies

To determine their specificity, expanded cultures (see above) were tested for their ability to bind five species or biotypes of Treponemes and six other bacteria. All typed or wild type oral microbes used to determine the specificity of monoclonal antibodies were obtained either from ATCC, or were isolated from local clinical samples and typed according to standard techniques and are listed below in Table 1. Monoclonal antibodies were also tested against LPS extracted from the following microbes by the method described in Westphal et al., Biochem. Rev. 9:191-195 (1981) and Westphal et al., Progress Allergy 33:9-39 (1983), the dislosures of which are hereby incorporated by reference: A. actinomycetumcommitans, B. gingivalis, B. intermedius, E. corrodens, F. nucleatum, h. aphrophilus, T. denticola, T. vincentii. Extracted LPS from E. coli (LPS 055:BS, 0127B8 and 0111:B4), Pseudomonas aeruginosa (F-D, type 1) and Salmonella minnesota (Re595) were obtained from Calbiochem, San Diego, CA. Extracted LPS from Vibrio cholerae was obtained from Sigma Chemical Co., Purified Lipid A (from S. minnesota) was obtained from Calbiochem. Lipid A was also acid extracted and purified from B. gingivalis by the methods known to those skilled in the art.

All bacteria were crosslinked to microtiter plates using gluteraldehyde as above. Supernatants from hybridoma cultures were added and binding was detected using goat anti-mouse Ig as described in Example 1. Table 2 describes the results of those experiments for forty-five antibodies binding T. denticola (designated "TDE", or alternatively "TDF", herein) and twelve antibodies binding T. vincentii ("TVC"). The number after the TDE (or TDF) or TVC designation indicates the clone number, and the number after the hyphen, if present, indicates the number of the sub-clone. In Table 2, a "+" sign indicates a positive immunologic reaction was detected between the indicated monoclonal antibody and bacterial strain, a "-" indicates that no such reaction was detected, and "ND" indicates that no data exists for that particular reaction.

## Table 1

| STRAINS | SOURCE (ATCC Nos., Clinical Isolates (see footnote)) |
|---|---|
| Actinobacillus actinomycetumcommitans | 29522, 29523, 29524 |
| Bacteroides buccae | 33574 |
| Bacteroides fragilis | 25285 |
| Bacteroides gingivalis | 33277 |
| Bacteroides intermedius | 25261 |
| Bacteroides loeschii | 15930 |
| Bacteroides melaninogenicus | 15032, 15033 |
| Bacteroides oris | 33573 |
| Capnocytophaga ochracea | (2) |
| Capnocytophaga sputigina | 33612 |
| Eikenella corrodens | 23834, (2) |
| Escherichia coli | 4157 |
| Eubacterium limosum | 8486 |
| Fusobacterium nucleatum | 25586, (1) |
| Haemophilus aphrophilus | 13232 |
| Haemophilus paraphrophilus | (1) |
| Haemophilus segnis | 7901 |
| Pasteurella multocida | 6530 |
| Shigella sonni | 9290 |
| Streptococcus pyogenes | 7958 |
| Treponema denticola | 33520, 33521, 35404, 35405, (3) |
| Treponema pallidum | 27087 |
| Treponema pectinovorum | 33768 |
| Treponema phagedenis | (3) |
| Treponema scoliodontum | (3) |
| Treponema socranskii | (3) |

Clinical isolate sources:

(1) Dr. L. F. Wolff, University of Minnesota, School of Dentistry

(2) Dr. W. F. Liljemark, University of Minnesota, School of Dentistry

(3) Dr. R. C. Johnson, University of Minnesota, Department of Microbiology

## Table 2

BINDING OF MONOCLONAL ANTIBODIES TO VARIOUS BACTERIA

| Clone Number | BINDING TO BACTERIA | | | | | | | | | | |
|---|---|---|---|---|---|---|---|---|---|---|---|
| | A | B | C | D | E | F | G | H | I | J | K |
| TDE01-01 | + | − | − | − | − | − | − | − | − | − | − |
| TDE02 | + | − | − | − | − | − | − | − | − | − | − |
| TDE03 | + | − | − | − | − | − | − | − | − | − | − |
| TDE04 | + | − | − | − | − | − | − | − | − | − | − |
| TDE05 | + | − | − | − | − | − | − | − | − | − | − |
| TDE06 | + | − | − | − | − | − | − | − | − | − | − |
| TDE07 | + | − | − | − | − | − | − | − | − | − | − |
| TDE08 | + | − | − | − | − | + | − | − | − | − | − |
| TDE09 | + | + | − | − | − | − | − | − | − | − | − |
| TDE10 | + | − | − | − | − | − | − | − | − | − | − |
| TDE11 | + | − | + | + | − | − | − | − | − | − | − |
| TDE12 | + | + | + | + | + | + | + | + | + | + | − |
| TDE13 | + | − | − | − | + | − | − | − | − | − | − |
| TDE18 | + | − | − | − | − | − | − | − | − | − | − |
| TDE21 | + | + | + | − | + | − | − | − | − | − | − |
| TDE23 | + | + | + | − | + | + | − | − | − | − | − |
| TDE24 | + | − | + | − | + | − | − | − | − | − | − |
| TDE25 | + | − | − | − | − | − | − | − | − | − | − |
| TDE26 | + | − | + | + | + | + | − | − | − | − | − |
| TDE28 | + | + | + | + | + | + | + | + | + | + | − |
| TDE29 | + | + | + | + | + | + | + | + | + | + | − |
| TDE30 | + | + | + | − | + | + | + | + | + | + | − |
| TDE32 | + | − | − | − | − | − | − | − | − | − | − |
| TDE37 | + | − | − | − | − | − | − | − | − | − | − |
| TDE38 | + | + | − | − | − | − | − | − | − | − | − |
| TDE42 | + | − | − | − | − | − | − | − | − | − | − |
| TDE47 | + | − | + | − | + | − | − | − | − | − | − |
| TDE51 | + | + | + | + | + | + | − | − | − | − | − |
| TDE54-04 | + | + | + | + | + | − | − | − | − | − | − |
| TDE58 | + | + | − | − | + | − | − | − | − | − | − |
| TDE62 | + | − | − | − | − | − | − | − | − | − | − |

Table 2 (cont.)

BINDING OF MONOCLONAL ANTIBODIES TO VARIOUS BACTERIA

| Clone Number | BINDING TO BACTERIA | | | | | | | | | | |
|---|---|---|---|---|---|---|---|---|---|---|---|
| | A | B | C | D | E | F | G | H | I | J | K |
| TDE68 | + | + | + | − | + | − | − | − | − | − | − |
| TVC01 | + | − | + | − | − | − | − | − | − | − | − |
| TVC02-01 | − | − | + | + | − | − | − | − | − | − | − |
| TVC04 | − | − | + | + | − | − | − | − | − | − | − |
| TVC07 | − | − | + | + | − | − | − | − | − | − | − |
| TVC09 | − | − | + | + | + | − | − | − | − | − | − |
| TVC15 | − | − | + | + | − | − | − | − | − | − | − |
| TVC17 | + | + | + | + | + | + | ND | ND | ND | ND | ND |
| TVC19 | + | + | + | − | − | − | ND | ND | ND | ND | ND |

A = Treponema denticola-biotype I;

B = Treponema denticola-biotype II;

C = Treponema vincentii;

D = Treponema scoliodentum;

E = Treponema phageadenis (Riter biotype);

F = Bacteriodes gingivalis;

G = Salmonella typhimurium;

H = Pasteurella multocida;

I = Escherichia coli;

J = Shigella sonni;

K = Streptococcus pyogenes

The data in Table 2 indicate that monoclonal antibodies can be obtained that are specific for a particular T. denticola biotype (e.g., TDE 01), or for the T. denticola species as a whole as opposed to T. vincentii (e.g., TDE 09), or for T. vincentii as opposed to T. denticola (e.g., TVC 01). Hybrid cell lines producing monoclonal antibodies to T. denticola and T. vincentii have been deposited in the permanent collection of the American Type Culture Collection, 12301 Parklawn Drive, Rockville, MD, USA 20852, and assigned ATCC Accession Nos. (Applicant's designation Nos. TDF01F49 and TVC02E14-01, deposited November 18, 1986), respectively.

Example 3

Antibody Binding to Mammalian Cells

It has been reported that some spirochete species can induce antibodies to antigens of mammalian origin. Since reactivity of this nature could produce undesirable background in an immunoassay if the sample contained such mammalian antigens, it was important to determine if this were the case for any of the antibodies constructed.

Hep-2 cells (human epitheloid tumor cells, American Type Culture Collection No. CCL 23, Rockville, MD) were grown on glass slides, fixed in acetone and air dried. Culture fluids from hybridomas producing antibodies to spirochetes were placed atop the cells, incubated for one hour, and washed. Fluorescent goat anti-mouse IgG (Cappel Laboratories, Cockneyville, PA) was then added, incubated for an additional hour and washed to remove unbound antibody. The cells were mounted in phosphate buffered glycerol and examined with an inverted fluorescent microscope. Antibodies binding to those cells were then eliminated from further consideration for microbial assays.

### Example 4

#### Antibody Binding to Bacterial Cellular Constituents

To further characterize the binding of the antibodies to the spirochetes they were examined by "Western Blots" according to the method of Tobin et al., Proc. Nat. Acad. Sci. 76:4350 (1979) the disclosure of which is hereby incorporated by reference. In this method the constituent molecules of the microbes are denatured, reduced and separated by polyacrylamide gel electrophoresis. The separated molecules segregate by molecular weight. These are then quantitatively transferred to nitrocellulose paper which binds them in a manner suitable for probing by the monoclonal antibodies. The binding of the antibodies to the immobilized antigen is detected with an enzyme-labeled goat anti-mouse IgG, and indicates the molecular weight of the antigen. The specificity of the antibodies for their respective antigens is thereby established.

Whole washed spirochetes (T. denticola or T. vincentii) were dissolved in a minimum amount of electrophoresis buffer (4% sodium dodecyl sulfate ("SDS", BioRad Laboratories, Richmond, CA); 10% 2-mercaptoethanol, 20% glycerol, 0.125M Tris pH 6.8) and placed in a boiling water bath for 2 minutes. Samples of each were electrophoresed in 10% polyacrylamide gels at 25 mA for 2-3 hours until a dye marker cleared the gel. The gel was placed on a square of nitrocellulose (size 15 × 15 cm, BioRad Laboratories) and transferred in tris-methanol buffer overnight at 30 mA according to the method of Laemmli, U.K., Nature 227:680-685 (1970), the disclosure of which is hereby incorporated by reference. The transferred antigens were washed in phosphate buffered saline containing 0.05% "Tween 20" (Fisher Diagnostics, Orangebury, NY) surfactant ("PBS-Tween 20"), pH 7.4 and placed in 3 ml of antibody-containing supernatant. After 1 hour at room temperature the antigens were washed to remove unbound antibodies and covered with 3 ml of peroxidase labeled goat anti-mouse Ig. After another one hour incubation and an additional wash the blots were transferred to peroxidase substrate solution to develop the reaction. The number and location of the bands that developed was noted and compared with molecular weight standards.

The Western Blots indicated that clone TDE 01-01 produced a monoclonal antibody that did not bind on the blot, infering that it binds to an antigen that was denatured by the Western blotting technique. TDE 54-04 bound to a 67,000 dalton molecular weight species, which is a common Treponeme antigen, and apparently is not LPS. TVC 02-01 bound a variety of molecular weight species in a manner consistent with the pattern to be expected of LPS.

### Example 5

#### Antibody Binding to Treponemal Outer Envelope

In addition to the normal cell wall and membrane structures of most gram-negative bacteria, members of the order Spirocheatales possess an outer envelope. Many of the antigens associated with these organisms may be contained in this structure. The outer envelope can be selectively removed from the organisms by detergents (Johnson et al., Infect. Immun. 7:249-258 (1973)), then separated from other components by differential centrifugation. Outer envelope preparations from T. denticola and T. vincentii were adsorbed to microtiter plates and tested for their ability to bind antibody. Outer envelope preparations were diluted in carbonate buffer (0.1 M Na bicarbonate; 0.1 M Na carbonate, pH 9.5) to 20μg/ml, and 50μl aliquots pipetted into each microtiter well. After overnight incubation at 4°C the plates were washed with PBS-Tween 20, and 50μl of antibody solutions (TDE 54-04 and TVC 02-01) of various concentrations were added. After incubation for 1 hour at room temperature the plates were washed and 50μl of peroxidase labeled goat anti-mouse Ig was added. The plates were incubated for 1 hour at room temperature, washed with PBS-Tween 20, and 10μl of peroxidase substrate solution was added. The antibody binding was detected using a spectrophotometric plate reader at 490 nm. The results are listed in Table 3.

### Table 3

| Clone # | Outer Envelope | |
|---------|----------------|----------------|
|         | T. denticola | T. vincentii |
| TVC01-01 | — | — |
| TDE01-01 | — | — |
| TDE54-04 | + | + |

TDE54-04 clearly binds a common Treponeme antigen on the outer envelope of both microbes.

## Example 6

### Immunofiltration with Treponemes

Treponemes fall at an inconvenient size for the clear cut use of filtration assays. While the organisms are long (6-20μm) they have relatively small diameters (0.15-0.25μm), a fact that is used to separate them from other bacteria in primary isolation. It was necessary, therefore, to determine the longest membrane pore size (to facilitate washing the clinical samples) that would retain the spirochetes. Membrane cutoffs available were: 0.10μm; 0.22μm; 0.45μm; and 5.0μm. Ten million T. denticola organisms in PBS-Tween 20 were added to each filter. The microbes were immobilized by pulling the suspension through the filter with vacuum. The suspensions were washed 2 times with PBS-Tween 20 after which antibody to the spirochetes was added. After 1 hour incubation the plates were washed again and peroxidase labeled goat anti-mouse IgG added. After an additional incubation and wash the enzyme substrate was added (100μl) and the optical density at 490nm determined. Surprisingly, the 0.45μm filter retained the spirochetes with the same efficiency as the 0.22 m filters. Filters of 0.10μm and 5μm were judged unsuitable.

To determine the best pore size to facilitate washing clinical materials, twenty-four plaque samples spiked with varying amounts of microbes were placed in microtiter wells with 0.45μm and 0.22μm filters ("Millititer ", Millipore Co., Bedford, MA). The samples were pulled onto the filters as described above and washed. The filters were observed for signs of plugging. The 0.22μm filters plugged with all but the lowest levels of microbes, whereas the 0.45μm filters retained their ability to pass buffer after loading with all plaque samples. Goat anti-mouse IgG was added to each of the cells, washed 3 times and peroxidase substrate solution was added to determine non-specific background levels associated with the immobilized plaque. All wells with 0.22μm filters proved unsatisfactory, i.e., had high background. All wells with 0.45μm filters were determined to give only slight or no background. The slight background levels found in some wells were not associated with the number of microbes immobilized in the wells (coefficient of correlation = 0.089).

To determine the usefulness of the immunofiltration assay with spirochetes, $10^6$ bacteria in 100μl of PBS-Tween 20 were incubated 15 minutes at room temperature with 100μl of various dilutions of anti-spirochete monoclonal antibodies. The mixture was then added to wells in 0.45μm Millititer plates and washed 3 times with PBS-Tween 20. A 50μl aliquot of goat anti-mouse IgG was added and allowed to incubate for 15 minutes at room temperature. The plates were washed 3 times as above, 100μl of peroxidase substrate solution was added and the optical density at 490 nm was measured. Dilution curves plotting OD490 vs. log bacterial concentration were obtained that indicated that the assay exhibited optimal sensitivity between about $5 \times 10^3$ and $1.5 \times 10^5$ bacteria per sample.

## Example 7

### Immunofiltration Assay with Spiked Plaque Samples

Plaque samples from individuals without detectable periodontal disease were obtained by the Listgarten et al. (J. Periodontol. 8:122 (1981)) method and were spiked with various numbers of T. denticola and T. vincentii. One hundred μl of anti-spirochete monoclonal antibodies were added and the samples incubated 1 hour at room temperature. The materials were transferred to wells in 0.45μm Millititer plates and immobilized as in Example 6. The immobilized plaque was washed 3 times with PBS-Tween 20 and 100μl of peroxidase labeled goat anti-mouse IgG added. After an additional half hour incubation the unbound labeled antibody was removed and the wells washed 3 times as above. A 100μl aliquot of peroxidase substrate solution was added and the optical density at 490 nm determined. Correlation coefficients between the number of spirochetes in spiked samples as determined by the optical density and the number of spirochetes as determined by phase-contrast microscopy was determined to be 0.873, i.e., well within acceptable limits.

## Example 8

### Detection of Spirochetes in Plaque Samples

Five hundred and twenty four plaque samples were obtained using the method of Listgarten et al. and evaluated for the number of spirochetes. These samples were then tested in an immunofiltration assay using the TDE 01-01 antibody described above.

Plaque samples were immobilized in Microtiter plates with membrane filters and washed 3 times with with PBS-Tween 20. Monoclonal antibody TDE 01-01 (50μl) was added to each well and incubated at room temperature for 1 hour. After additional washes as above, peroxidase-labeled goat anti-mouse IgG was added (50μl) and incubated for an additional hour. The wells were washed again and enzyme substrate was added. The optical density of each well was determined and samples were scored positive when the O.D. exceeded that of a group of known negative samples (mean $OD_{490}$ + 2 standard deviations). The results shown in Table 4 below, indicate that the immunofiltration assay provides results that correlate well with the much longer and more laborious process of dark-field microscopy.

## TABLE 4

| | POSITIVE | | | NEGATIVE | | |
|---|---|---|---|---|---|---|
| | Dark Field | Immuno-assay | False Negative (Immunoassay) | Dark Field | Immuno-assay | False Positives (Immunoassay) |
| Good Oral Health | 29 | 23 (79%) | 6 (21%) | 451 | 431 (95%) | 20 (5%) |
| Mild-Severe Periodontitis | 33 | 28 (85%) | 5 (15%) | 11 | 9 (82%) | 2 (18%) |
| Total | 62 | 51 (82%) | 11 (18%) | 462 | 440 (95%) | 22 (5%) |

Example 9

Monoclonal antibodies were prepared as described above that were specific for each of the following species: Actinobacillus actinomycetumcommitans, Bacteroides gingivalis, Bacteroides intermedius, Fusobacterium nucleatum, and Haemophilus aphrophilus. The bacterial sources, and ATCC accession numbers for hybidoma cell lines producing such antibodies are set forth below in Table 5. Monoclonal antibodies to each species having a specificity for LPS were determined by each of three methods described above:

    1) immunoreactivity with known strains, wild type strains, extracted LPS and Lipid A as described in Example 1, by either an immunofiltration assay as described in Example 6 or by particle counting fluorescent immunoassay using a "Pandex" immunoassay plate, (Catalogue No. 22-010-1, Pandex Laboratories, Inc., Chicago, IL) as described in Dawson, M., Research Report No. 6, July , 1984, Pandex Laboratories, Inc., and Masson, P.L. et al., in Methods in Enzymology, Langone et al., eds., Vol 74:106-139 (1981) the disclosures of which are hereby incorporated by reference;

    2) Western blot analysis of whole cell lysates; and

    ) ELISA immunoassay as described in Example 1 using purified LPS.

## TABLE 5

| Oral Microbe | Source | Hybridoma Accession Number |
|---|---|---|
| A. actinomycetumcommitans | ATCC 29522 | (ACT04F02, deposited 11/18/86) |
| B. gingivalis | ATCC 33277 | (BGN55F43, deposited 11/18/86) |
| B. intermedius | ATCC 25261 | (BNT06G07, deposited 11/18/86) |
| F. nucleatum | ATCC 25586 | (FUS05F02, deposited 11/18/86) |
| H. aphrophilus | ATCC 13232 | (HAP02F4S, deposited 11/18/86) |

Example 10

Monoclonal antibodies were prepared and selected to be immunologically reactive with all gram-negative oral microbes evaluated. Such antibodies were prepared as described above using B. gingivalis (ATCC 33277) as the immunogenic microbe. A hybridoma cell line producing such antibodies has been deposited and assigned ATCC Accession No. (Applicant's designation BGN58F43, deposited November 18, 1986).

The monoclonal antibody produced by this cell line was found to be cross-reactive with every gram-negative oral microbe tested, including the typed strains, wild type strains, extracted LPS and Lipid A described in Example 1.

The epitope to which this monoclonal antibody reacts was determined to be LPS by Western Blot analysis as described above.

Example 11

Monoclonal antibodies described above were used to correlate the method of this invention with both a clinical assessment of periodontal disease in 20 patients and an assessment of the microbial flora by anaerobic culture techniques in those same patients.

Plaque samples were obtained as described and dispersed in prereduced anaerobically sterilized ("PRAS") diluent, prepared as described below, and diluted 1:100 in sterile PBS. One-half milliliter of the diluted sample was then spread on the surface of a 150mm culture plate using a sterile glass policeman. Anaerobe Medium I (DiMed, Inc., Minneapolis, Minnesota) was used to culture Actinobacillus, Bacteriodes, and Fusobacterium. Modified Chocolate Agar (DiMed, Inc., Minneapolis, Minnesota) was used for culturing Haemophilus. Anaerobic Medium I plates were incubated 10 days anaerobically at a temperature of 37°C. Chocolate Agar plates were incubated 3-4 days under 10% $CO_2$ (90% air) at a temperature of 37°C. All confirmation of species was done in the manner described in Wolff et al., J. Perio. Res. 20:237-250 (1985) the disclosure of which is hereby incorporated by reference. Treponemes were not run due to the difficulty encountered in culturing these microbes.

PRAS diluent was prepared in the following manner:

Add slowly to 200 ml water;

    0.2 g Ca $Cl_2$
    0.2 g $MgSO_4.7H_2O$

Add slowly to 500 ml water;

    1.0 g $K_2HPO_4$
    1.0 g $KH_2PO_4$
    2.0 g NaCl
    10.0 g $NaHCO_3$

Combine both solutions, and add 200 ml water. Take 400 ml of the combined solutions, and add

    400 ml water
    1.6 g gelatin (Difco, Detroit, MI)
    3.2 ml oxygen indicator (0.025% Resazurin®, Difco)
    0.4 g cysteine HC1

Adjust pH to 6.6 with concentrated HC1. Place solution in anaerobic hood for 24 hours, and aseptically dispense 20 ml in to each of several sterile pharmacy bottles and cap.

Samples from 20 patients were colony blotted as described in McArthur et al, J. Clin. Periodontol. 13:684-690 (1986), and were also run by immunofiltration assay as described in Example 6. The proportion of each of the five oral microbes tested in the colony blot assay is expressed as a percent of the total LPS-containing cells, as calculated by the formula:

$$\frac{\text{Number of specific LPS colonies}}{\text{Number of total LPS - positive colonies}} \times 100\%$$

For each antibody these proportions were determined by each method (i.e., colony blotting and immunofiltration) and the two proportions were plotted against each other. A best fit curve was determined by linear regression analysis. The correlation coefficient between these values was then determined according to the method of Piazza et al., Immunological Methods (Lefkovits et al., eds.) Vol. I, pp. 419-456 (1979), and is set forth below in Table 6.

## Table 6

| Oral microbe | Correlation coefficient colony blot vs. immunoassay |
|---|---|
| A. Actinomycetumcommitans | .771 |
| B. gingivalis | .794 |
| B. intermedius | .850 |
| H. aphrophilus | .814 |
| F. nucleatum | .693 |

These results indicate that the method of the present invention correlates very well ($P \geq 0.005$) with a traditional cultivation method for the identification and comparison of oral microbes.

Various modifications and alterations will be apparent to those skilled in the art without departing from the scope and spirit of this invention, and it should be understood that this invention is not to be unduly limited to the illustrative embodiments set forth herein.

## Claims

1. A method for evaluating an oral sample for the presence of microbial flora associated with periodontal disease, characterized in that said method comprises the steps of:
   (a) determining, by the use of antibodies, the amount in said sample of one or more specific oral microbe(s),
   (b) determining, by the use of antibodies, the amount in said sample of a reference group of gram-negative oral microbes, and
   (c) comparing the amount of said specific oral microbe(s) to the amount of said reference microbes.

2. A method according to claim 1 wherein said specific oral microbes are selected from the group consisting of the genera Actinobacillus, Bacteroides, Fusobacterium, Haemophilus, and Treponema and said antibodies of step (a) are monoclonal antibodies.

3. A method according to claim 2 further characterized in that said specific oral microbes are selected from the group consisting of the species Actinobacillus actinomycetumcommitans, Bacteroides gingivalis, Bacteroides intermedius, Fusobacterium nucleatum, Haemophilus aphrophilus, Treponema denticola and Treponema vincentii and said specific oral microbes are detected, respectively, by monoclonal antibodies produced by hybrid cell lines having ATCC Accession Nos. HB 9268, HB 9269, HB 9270, HB 9271, HB 9272, HB 9273 and HB 9274.

4. A method according to claim 2 further charaterized in that said monoclonal antibodies are specific for lipopolysaccharide.

5. A method according to claim 1 further characterized in that said reference microbes comprise all gram-negative microbes in said sample and the amount of said reference microbes is determined by the use of a single monoclonal antibody.

6. A kit for the evaluation of an oral sample for the presence of microbial flora associated with periodontal disease, characterized in that said kit comprises
   (a) one or more antibodies to specific oral microbes, and
   (b) one or more antibodies to a reference group of gram-negative oral microbes.

7. A kit according to claim 6 further characterized in that said specific oral microbes are selected from the group consisting of the genera Actinobacillus, Bacteroides, Fusobacterium, Haemophilus, and Treponema and said antibodies to said specific oral microbes and to said reference microbes are monoclonal antibodies.

8. A kit according to claim 7 further characterized in that said specific oral microbes are selected from the group consisting of the species Actinobacillus actinomycetumcommitans, Bacteroides gingivalis, Bacteroides intermedius, Fusobacterium nucleatum, Haemophilus aphrophilus, Treponema denticola and Treponema vincentii and said monoclonal antibodies to said specific oral microbes are produced by hybrid cell lines having ATCC Accession Nos. HB 9268, HB 9269, HB 9270, HB 9271, HB 9272, HB 9273 and HB 9274.

9. A kit characterized in that said kit comprises one or more monoclonal antibodies specific for one or more species of gram-negative oral microbes, and one or more further monoclonal antibodies specific for all gram-negative oral microbes.

10. A kit according to claim 6 further characterized in that said kit comprises enzyme-linked antibodies to said antibodies of part (a) and to said antibodies of part (b), and substrate for said enzyme of said enzyme-linked antibodies.